Europäisches Patentamt

European Patent Office

Office européen des brevets

(11) Publication number : **0 401 370 B1**

# EUROPEAN PATENT SPECIFICATION

(45) Date of publication of patent specification :
24.05.95 Bulletin 95/21

(51) Int. Cl.⁶ : **G01N 33/53, G01N 33/577**

(21) Application number : **89902540.7**

(22) Date of filing : **17.02.89**

(86) International application number :
**PCT/JP89/00161**

(87) International publication number :
**WO 89/07761 24.08.89 Gazette 89/20**

(54) **ENZYME IMMUNOASSAY ACCORDING TO SANDWICH METHOD OF HUMAN IV-TYPE COLLAGEN.**

(30) Priority : **19.02.88 JP 35099/88**

(43) Date of publication of application :
**12.12.90 Bulletin 90/50**

(45) Publication of the grant of the patent :
**24.05.95 Bulletin 95/21**

(84) Designated Contracting States :
**DE FR GB IT**

(56) References cited :
JP-A- 5 716 355
JP-A- 6 324 396
PROCEEDINGS OF THE NATL. ACADAMY OF
SCIENCES USA, vol. 81, December 1984; B.F.
ODERMATT et al., pp. 7343-7347
LABORATORY INVESTIGATION, vol. 48, no. 5,
1983, United States Canadian Division of the
Internatl. Academy of Pathology; H.G. FOELL-
MER et al., pp. 69-649
AMERICAN JOURNAL OF PATHOLOGY, vol.
108, no. 3, 1982, American Association of
Pathologists, US; L.Y. SAKAI et al., pp. 310-318
JOURNAL OF BIOCHEMISTRY, vol. 103, no. 5,
1988; J. KINO et al., pp. 829-835
CLINICA CHIMICA ACTA, vol. 181, 1989,
Elsevier Science Publishers B.V. (Biomedical
Devision), NL; K.-I. OBATA et al., pp. 293-304
EUROPEAN JOURNAL OF BIOCHEMISTRY,
vol. 108, no. 1, 1980; J. RISTELI et al., pp.
239-250

(56) References cited :
EUROPEAN JOURNAL OF BIOCHEMISTRY,
vol. 95, no. 2, 1979, R. TIMPL et al., pp. 255-263
EUROPEAN JOURNAL OF CLINICAL INVEST.,
vol. 9, 1979, ROHDE et al., pp. 451-459
CLINICAL CHIMICA ACTA, vol. 144, 1984;
HÖGEMAN et al., pp. 1-10
JOURNAL OF CLINICAL INVEST., vol. 78,
1986; SCHUPPAN et al., pp. 244-248

(73) Proprietor : **FUJI YAKUHIN KOGYO
KABUSHIKI KAISHA**
530, Chokeiji
Takaoka-shi Toyama 933 (JP)

(72) Inventor : **OBATA, Kenichi**
7-13, Honmachi
Tonami-shi
Toyama 939-13 (JP)
Inventor : **IWATA, Kazushi**
190, Ikarihigashimachi
Takaoka-shi
Toyama 933 (JP)
Inventor : **OSHIMA, Akira**
307, Ribahaitsuota
11, Funatsucho 1-chome
Wakayama-shi Wakayama 640 (JP)
Inventor : **INOUE, Kyoichi**
5-18, Nagaeshinmachi 1-chome
Toyama-shi Toyama 930 (JP)

(74) Representative : **VOSSIUS & PARTNER**
Postfach 86 07 67
D-81634 München (DE)

## Description

FIELD OF THE INVENTION

The present invention relates to a method for the quantitation of human type IV collagen by sandwich enzyme immunoassay useful for diagnosing liver disorders in a straightforward manner.

More particularly, the invention provides a method for the quantitation of the large central triple-helical region of human type IV collagen and a method for the quantitation of human type IV collagen 7-S domain, both by enzyme immunoassay based on the one step sandwich technique in which monoclonal antibodies against human type IV collagen are utilized.

BACKGROUND OF THE INVENTION

Radio immunoassays for human type III procollagen N-terminal peptide and human type IV collagen N-terminal peptide 7-S domain and C-terminal peptide NC1 domain in serum using corresponding polyclonal antibodies were already reported (Rohde et al., Eur. J. Clin. Invest., 9, 451-459, 1979; Högemann et al., Clin. Chim. Acta, 144, 1-10, 1984; Schuppan et al., J. Clin. Invest. 78, 244-248, 1986).

American J. Pathol. 108, 310-318(1982) and J. Biochem. 103, 829-835 (1988), the latter representing state of the art according to Art. 54(3) EPC only, disclose monoclonal antibodies directed against the triple helical region of type IV collagen.

However, nothing has been reported on how to quantitate the major central triple-helical domain of human type IV collagen and human type IV collagen 7-S domain in serum.

As a result of extensive researches made by the present inventors of methods for quantitating human type IV collagen in a specific manner, the present invention has now been successfully achieved according to which there is provided a method enabling the quantitation of human type IV collagen peptide to be performed in a precise and rapid manner with small amounts of samples, wherein an enzyme immunoassay based on the one step sandwich technique is carried out using monoclonal antibodies against pepsin-solubilized human type IV collagen or against human type IV collagen 7-S domain. The hitherto known sandwich technique involves the first reaction, i.e. reaction between solid phase-immobilized antibodies and antigens in samples, the second reaction, i.e. reaction between solid phase-immobilized antibody-antigen complexes and enzyme-labeled antibodies, and the third reaction, i.e. coloring reaction with enzyme substrates, thus requiring two immunological reaction steps prior to the coloring reaction. In contrast to such sandwich technique, the method according to the present invention relies on the one step sandwich technique in which the first and second reactions are effected at the same time, thus making it possible to straight forwardly perform assays of a large number of samples with greater precision within shorter periods of time.

DISCLOSURE OF THE INVENTION

The present invention provides a method for the quantitation of human type IV collagen by enzyme immunoassay based on the one step sandwich technique as described in (1) and (2) below:

(1) a method for the quantitation of the major central triple-helical region of human type IV collagen by enzyme immunoassay based on the sandwich technique using monoclonal antibodies against pepsin-solubilized human type IV collagen, in which

(a) a sample solution prepared by diluting a sample to be assayed with a solution in a buffer of an enzyme-labeled antibody obtained by labeling with an enzyme a monoclonal antibody which reacts with the major central triple-helical region of pepsin-solubilized human type IV collagen and

(b) an antibody-coated solid phase comprising a monoclonal antibody, bound to a solid phase carrier, which reacts only with pepsin-solubilized human type IV collagen

are used, and which comprises mixing the antibody-coated solid phase (b) with the solution (a) to cause an immunoreaction to occur among the human type IV collagen present in the said sample to be assayed, the said enzyme-labeled antibody and the antibody bound to the said solid phase carrier, isolating the solid phase carrier and measuring the enzyme activity bound to the solid phase thereby to quantitate the major central triple-helical region of human type IV collagen;

(2) a method for the quantitation of human type IV collagen 7-S domain by enzyme immunoassay based on the sandwich technique using monoclonal antibodies against human type IV collagen 7-S domain, in which

(a) a sample solution prepared by diluting a sample to be assayed with a solution in a buffer of an enzyme-labeled antibody obtained by labeling with an enzyme a monoclonal antibody which reacts with pepsin-solubilized human type IV collagen 7-S domain and

(b) an antibody-coated solid phase comprising a monoclonal antibody, bound to a solid phase carrier, which reacts only with human type IV collagen 7-S domain are used, and which comprises mixing the antibody-coated solid phase (b) with the solution (a) to cause an immunoreaction to occur among the human type IV collagen 7-S domain present in the said sample to be assayed, the said enzyme-labeled antibody and the antibody bound to the said solid phase carrier, isolating the solid phase carrier and measuring enzyme activity bound to the solid phase thereby to quantitate the human type IV collagen 7-S domain.

Employed as the antibody to be labeled with an enzyme in the method of this invention is an IgG fraction obtained by the fractionation of a material containing antibodies with ammonium sulfate and the subsequent purification on DEAE-Sephacel and Protein A affinity columns.

The method of the present invention includes such an embodiment wherein the monoclonal and polyclonal antibodies used may be their specific binding sites $F(ab')_2$ or Fab' as such.

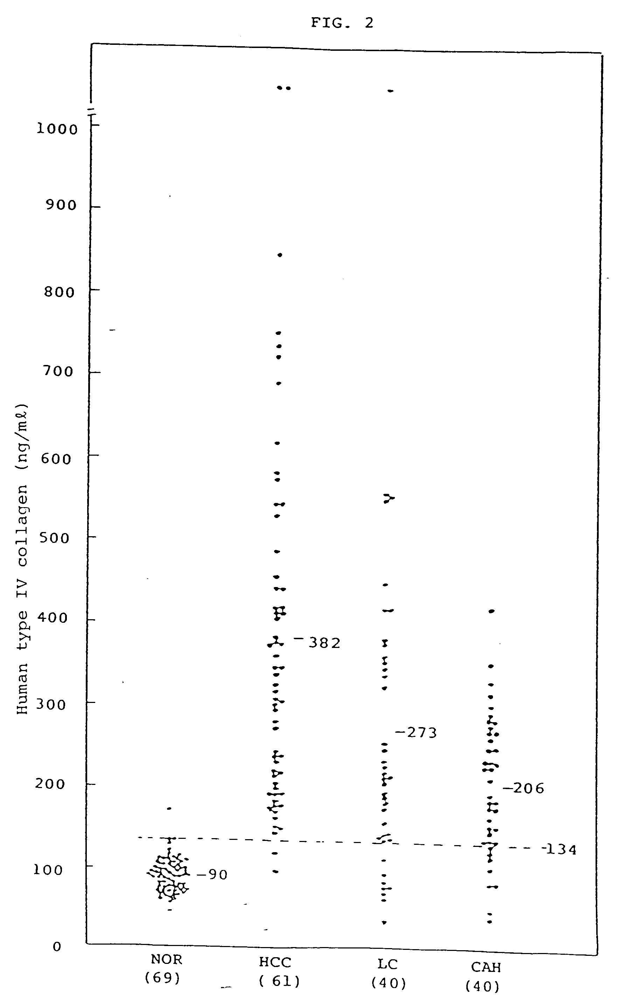

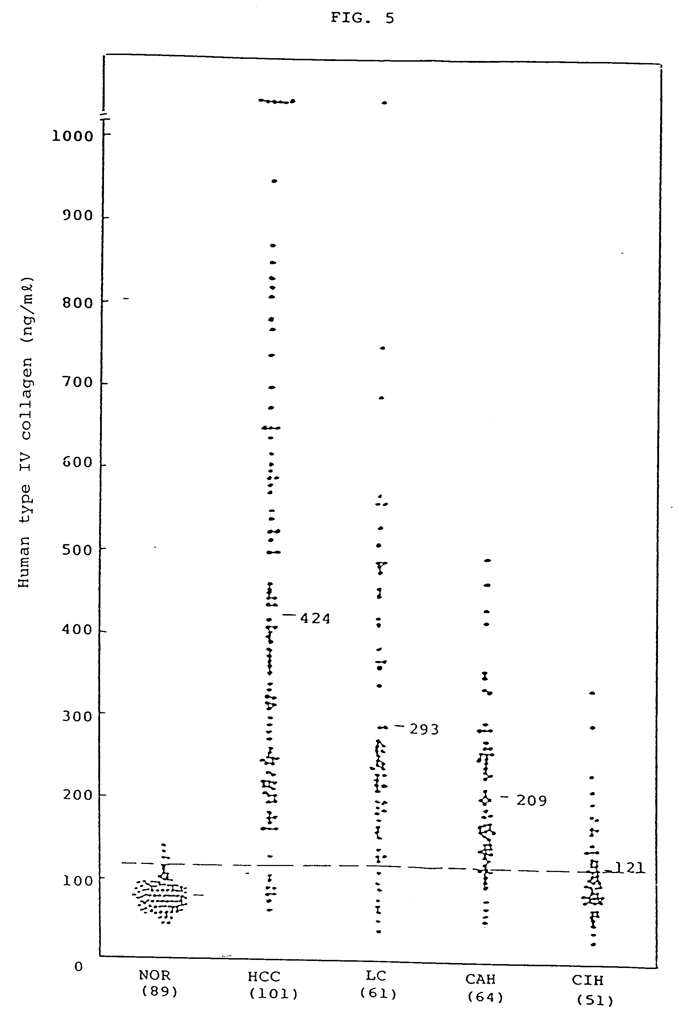

As will be shown in the accompanying Figs. 2 and 5, the levels of the major central triple-helical region of human type IV collagen peptide and of human type IV collagen 7-S domain in sera from patients with liver disorders as measured in accordance with the method of this invention are significantly higher than those in sera from healthy normal subjects. According to the method of the present invention, the measurement of levels of the major central triple-helical region of human type IV collagen peptide and of human type IV collagen 7-S domain in serum enables foreknowing liver disorders, especially liver fibrosis, hepatic carcinoma and digestive organ-metastatic liver carcinoma without relying on biopsy which is burdensome on patients.

We have confirmed that fibrosis of hepatic tissues, hepatic carcinoma, and digestive organ-metastatic liver carcinoma cannot be determined by the conventional liver function tests relying on ZTT (zinc sulfate turbidity test), GOT (glutamate-oxaloacetate transaminase), GPT (glutamate-pyruvate transaminase), ALP (alkaline phosphatase), LDH (lactate dehydrogenase), $\gamma$-GTP ($\gamma$-glutamyl transpeptidase), etc. Therefore, it is expected that detection of such disorders at an early stage is made possible by the measurement of serum levels of the major central triple-helical region of human type IV collagen peptide or of human type IV collagen 7-S domain in accordance with the method of the present invention in combination with the method for determining serum levels of human prolyl 4-hydroxylase previously reported by the present inventors (Japanese Laid-Open Patent Appln. No. Sho-61-202162).

The present invention is very useful since in accordance with the present method the diagnosis of fibrosis of hepatic tissues, hepatic carcinoma and digestive organ-metastatic liver carcinoma can be made based on the measurement of human type IV collagen peptide levels.

The present invention will now be illustrated in more detail by way of the following examples, but it is to be construed that the scope of this invention is not limited by these specific examples.

Example 1

Preparation of monoclonal antibodies against human type IV collagen peptide

(a) Preparation of pepsin-solubilized human type IV collagen as antigen:

According to the method of Mayne et al. [Artery, 7, 262-280, (1980)], human placenta was homogenized in 0.5 N acetic acid, digested with pepsin (1 mg/ml) to make the contained collagens soluble, and incorporated with NaCl so that its final concentration became 2 M to effect precipitation of the collagens. The precipitate was dissolved in 0.5 N acetic acid from which a fraction containing the type I and III collagens was obtained through dialysis against 0.5 N acetic acid solution containing 0.7 M NaCl and the supernatant was then dialyzed against 0.5 N acetic acid solution containing 1.2 M NaCl whereby a fraction containing the type IV and V collagens was precipitated. The fraction containing the type IV and V collagens was dissolved in 50 mM tris-HCl buffer (pH 7.4) containing 0.5 M NaCl and dialyzed against 50 mM tris-HCl buffer (pH 7.4) containing 2.2 M NaCl whereby the type IV collagen was precipitated and separated from the type V collagen. The purity of the type IV collagen thus obtained was determined to be about 95 % according to Sykes et al. method reported in Biochem. Biophys. Res. Commun., 72, 1472-1480 (1976) by subjecting the collagen to the sodium dodecyl sulfate-polyacrylamide gel electrophoresis (SDS-PAGE).

In this SDS-PAGE, there were also detected 11 different bands in the presence of mercaptoethanol: 174 KD, 135 KD, 108 KD, 92 KD, 78 KD, 68 KD, 60 KD, 56 KD, 43 KD, 34 KD and 29.5 KD.

(b) Preparation of different types of collagen, acid-extracted type IV collagen, type IV collagen 7-S domain, type IV collagen NC1 domain and laminin P1 fraction:

Using human placenta and newborn rat as source materials, the procedure as described in (a) above was followed. Pepsin-solubilized type IV collagen as well as type I, III, V and VI collagens was isolated and purified from human placenta. Also from new born rat was prepared pepsin-solubilized type IV collagen. Human type IV collagen N-terminal long 7-S domain was prepared, in accordance with the method reported by Risteli et al. in Eur. J. Biochem., 108, 239-250 (1980), from the pepsin-solubilized type IV collagen prepared in (a) above as starting material.

Acid-extracted type IV collagen was prepared from human placenta as source material in accordance with the procedure reported by Timpl et al. in Eur. J. Biochem., 84, 43-52 (1978) and 95, 255-263 (1979). Type IV collagen C-terminal NCl domain was prepared from the above-mentioned acid-extracted type IV collagen as starting material in accordance with the procedure reported by Timpl et al. in Eur. J. Biochem., 120, 203-211 (1981).

Laminin P1 fraction was prepared from human placenta as source material in accordance with the procedure reported by Risteli et al. in Biochem. J., 193, 749-755 (1981).

(c) Preparation of antibody-producing cells:

Two 8 weeks old BALB/c female mice were initially injected intraperitoneally with 100 µg of pepsin-solubilized human type IV collagen in Freund's complete adjuvant. After the initial administration, the mice were then forced to receive a booster injection of 100 µg antigen dissolved in 50 mM tris-HCl buffer (pH 7.4) containing 0.5 M NaCl, 2 to 4 times at 2 to 4 week intervals. As the final immunization, the mice received an intravenous administration 3 days before their spleens were extirpated and the spleen cells were prepared.

(d) Cell fusion Materials and methods used are as follows:

RPMI 1640 culture medium: RPMI 1640 (Difco Laboratories) supplemented with sodium bicarbonate (12 mM), sodium pyruvate (1 mM), L-glutamine (2 mM), penicillin G potassium (50 U/ml), streptomycin sulfate (50 µg/ml) and amikacin sulfate (100 µg/ml), adjusted at pH 7.2 with dry ice and filtered through a 0.2 µm Toyo membrane filter for sterilization.

NS-1 culture medium: the above RPMI 1640 culture medium supplemented with 15 % (v/v) fetal bovine serum (M.A. Bioproducts) filter-sterilized.

HAT culture medium: the above NS-1 culture medium further supplemented with hypoxanthine (100 µM), aminopterin (0.4 µM) and thymidine (16 µM).

HT culture medium: the same medium as the above HAT except that no aminopterin is contained.

PEG 4,000 solution: Serum-free RPMI 1640 culture medium, containing 50 % (w/w) polyethylene glycol 4,000 (PEG 4,000, Merck & Co., Inc.).

Tween, Sephacryl and Ultrogel are trade marks.

Utilizing 8-azaguanine-resistant myeloma cell line, NS-1 (P3-NS1-1), cell fusion was effected according to a slightly modified method of Oi et al. described in "Selected Method in Cellular Immunology" (ed. B. B. Mishell and S. M. Shiigi, W. H. Freeman and Company, (1980), pp. 351-372). The karyo-splenocytes prepared as described in the preceding (c) (cell viability 95%) were fused in a ratio of 5-6:1 with myeloma cells (cell viability 95%). Thus, the splenocytes and the myeloma cells were separately washed in RPMI 1640 culture medium, then suspended in the same medium and mixed together for fusion in the ratio described above. 40 ml of the RPMI 1640 culture medium was put into a 50 ml conical styrene resin test tube (Iwaki Glass), and centrifuged at 400xg for 10 min., and the supernatant was discarded completely by suction. To the precipitated cells was added dropwise over 1 min. 1 ml of the PEG 4,000 solution warmed at 37°C with gentle stirring. The cells were resuspended and dispersed by stirring for another minute. To the suspension was then added dropwise over 1 min. 1 ml of RPMI 1640 culture medium warmed at 37°C. After repeating the same operation once more, the cells were dispersed by adding 7 ml of the same culture medium dropwise over 2 to 3 min. under continuous agitation. The dispersion was centrifuged at 400xg for 10 min., and the supernatant was discarded completely by suction. To the precipitated cells was added rapidly 10 ml of NS-1 culture medium warmed at 37°C, and big cell clumps were then dispersed by pipetting carefully with a 10 ml pipette. The dispersion was diluted with 20 ml of the same culture medium, and distributed into 96-well microwell made of polystyrene (Iwaki Glass) so that $5.9 \times 10^5$ cells/0.1 ml exist in each well. As a preliminary treatment, the 96-well microwells had been treated with 0.2 ml of NS-1 culture medium overnight at 37°C in a $CO_2$ incubator, and the culture medium had been removed therefrom by suction on actual use. After completion of the cell fusion, the microwells were incubated at 37°C in 7 % $CO_2$/93 % air under 100 % humidity.

(e) Selective proliferation of hybridomas with the aid of a selective culture medium:

On the first day after the cultivation, two drops (ca. 0.1 ml) of HAT culture medium were added with a Pasteur pipette. On the 2nd, 3rd, 5th, 8th and 11th days, the half (0.1 ml) of each culture medium was replaced with fresh HAT culture medium. On 14th day, the half of each culture medium was replaced with fresh HT culture medium. Thereafter, the same operation was repeated at 3 to 4 days intervals. Usually, sufficient growth of hybridoma was observed in 2 to 3 weeks. All the wells containing hybridomas grown were tested to see if there are positive wells, using a solid phase-antibody binding test (ELISA) as will be described in the subsequent (f). All the cultures with hybridomas positive in the test above were transferred to a 24-well cell well made of polystyrene (Iwaki Glass) containing 1 ml of the HT culture medium containing $10^7$ mouse thymocytes as feeders. The cultures were incubated, as described in the preceding (d), at 37°C in 7 % $CO_2$ for about a week. Once or twice during the cultivation period 0.5 ml of the supernatant of each well was replaced with 0.5 ml of fresh HT culture medium. At the time the sufficient growth of hybridomas was observed, the cultures were checked again for the positivity by ELISA method, and subjected to a cloning procedure by the limiting dilution method as will be explained in (g) below. The residual culture after cloning was transferred into a 25 cm² tissue culture flask made of polystyrene (Iwaki Glass), for the preparation of a frozen sample for storage.

(f) Screening for hybridomas capable of producing anti-human type IV collagen peptide antibody by ELISA:

A method used for the present example was a slight modification of the method of Rennard et al. described in Anal. Biochem., 104, 205-214, (1980) which is appropriate for the detection of antibody production in hybridoma. Each well of a 96-well microtitration plate (Flow Laboratories, Inc.) was coated with 0.5 - 1.0 μg of pepsin-solubilized human type IV collagen and the uncoated part in each well was blocked with 1 % bovine serum albumin (BSA). To the wells was added a part of the supernatant from the wells containing hybridomas grown. The incubation was carried out at room temperature for about 1 hr. After the addition of goat anti-mouse immunoglobulin (Cappel Lab.) labeled with horseradish peroxidase (POD) as a secondary antibody, further incubation was carried out at room temperature for about 1 hr. The depth of brown color produced upon the addition of $H_2O_2$ and o-phenylenediamine (OPD) as a substrate was determined by measuring the absorbance at 492 nm using a microplate reader (MPR-A4, Toyo Soda Kogyo).

(g) Cloning:

In order to obtain hybridomas capable of producing monoclonal antibodies it is necessary for cloning to be carried out according to the limiting dilution method, since each well can develop two or more kinds of hybridomas. A cloning culture medium containing in NS-1 culture medium $10^7$ cells per ml of mouse thymocytes as feeders was prepared. 5, 1 and 0.5 cells/well of hybridomas were added to 36, 36 and 24 wells of a 96-well microwell, respectively. About 0.1 ml of NS-1 culture medium was further added to each well on the 5th and 12th day. About 14 to 15 days after the cloning when the sufficient growth of hybridoma could be recognized, the culture group in which more than 50 % wells were negative for colony formation was subjected to ELISA. When not all of the tested wells were shown to be positive, the colonies in antibody-positive wells were enumerated and out of them 4-6 wells exhibiting the formation of one and single colony were selected for recloning. Finally, 22 clones capable of producing monoclonal antibody to pepsin-solubilized type IV collagen were obtained.

(h) Proliferation *in vitro* and *in vivo* of monoclonal antibody:

Monoclonal antibodies can be obtained from the supernatant of an appropriate culture medium such as NS-1 culture medium in which each of the clones obtained in (g) was subjected to cultivation (*in vitro* proliferation) (The concentration of the monoclonal antibody protein 10 - 100 μg/ml). If a large amount of antibody is to be produced, on the other hand, an *in vivo* proliferation is carried out as described below. A carcinogenic excitant, Pristane (2, 6, 10, 14-tetramethylpentadecane, Aldrich Chemical), was administered to animals (Balb/c mice) intraperitoneally in a volume of 0.5 ml per animal, which animals were of the syngenic strain as those providing the splenocytes and the myeloma cells. One to 3 weeks after the administration, 1 x $10^7$ cells of each hybridoma were administered intraperitoneally as above, and ascites containing monoclonal antibody protein in a concentration of 4 - 7 mg/ml were obtained after 1 to 2 weeks.

(i) Isotypes of heavy and light chains of monoclonal antibodies:

First of all, each of the ascites obtained in (h) above was placed in a separate row of wells of a microtitration plate coated with pepsin-solubilized human type IV collagen, and the monoclonal antibody present in each ascite was bound thereto according to the above-described ELISA method. After washing, isotype-specific rabbit anti-mouse Ig antibodies (Zymed Laboratories) were added thereto. After washing, POD-labeled goat anti-rabbit IgG (H + L) antibody was added thereto, and 2,2'-azino-di(3-ethylbenzothiazoline sulfate-6) as a substrate and hydrogen peroxide were then used for detection isotypes of heavy and light chains. The results are shown in Table 1. Out of the resultant monoclonal antibodies against pepsin-solubilized human type IV collagen, 16 antibodies were found to contain immunoglobulin chain γ1/κ, 2 antibodies γ2b/κ, an antibody α/κ and 3 antibodies μ/κ.

## Table 1

| Clone No. obtained in (g) | Isotype | Chain |
|---|---|---|
| 4C1 | IgM | $\mu/\kappa$ |
| 7A11 | IgM | $\mu/\kappa$ |
| 1D3 | IgG1 | $\gamma 1/\kappa$ |
| 1D6 | IgG1 | $\gamma 1/\kappa$ |
| 1E10 | IgG1 | $\gamma 1/\kappa$ |
| 2A7 | IgM | $\mu/\kappa$ |
| 2D5 | IgG1 | $\gamma 1/\kappa$ |
| 2H1 | IgA | $\alpha/\kappa$ |
| 3A9 | IgG1 | $\gamma 1/\kappa$ |
| 4B1 | IgG1 | $\gamma 1/\kappa$ |
| 4H12 | IgG1 | $\gamma 1/\kappa$ |
| 5D10 | IgG1 | $\gamma 1/\kappa$ |
| 5F6 | IgG1 | $\gamma 1/\kappa$ |
| 6B5 | IgG1 | $\gamma 1/\kappa$ |
| 6C11 | IgG1 | $\gamma 1/\kappa$ |
| 6G5 | IgG2b | $\gamma 2b/\kappa$ |
| 7C8 | IgG1 | $\gamma 1/\kappa$ |
| 7H2 | IgG1 | $\gamma 1/\kappa$ |
| 8B4 | IgG1 | $\gamma 1/\kappa$ |
| 8G12 | IgG1 | $\gamma 1/\kappa$ |
| 9A3 | IgG2b | $\gamma 2b/\kappa$ |
| 9C7 | IgG1 | $\gamma 1/\kappa$ |

(j) Purification of monoclonal antibody:

Antibodies in the ascites obtained in the foregoing (h) were fractionated with ammonium sulfate (40 % saturation). IgG class was loaded on DEAE-Sephacel (Pharmacia) equilibrated with 40 mM phosphate buffer (pH 8.0) containing 0.06 M NaCl to separate an unadsorbed fraction and the fetal calf serum from culture medium and mouse-derived proteins were separated and removed.

For purification of IgA and IgM classes, they were subjected to column chromatography on DEAE-Sephacel equilibrated with 40 mM phosphate buffer (pH 8.0) containing 0.1 M NaCl for elution of the respective fractions with a NaCl gradient from 0.1 M to 1.0 M.

The IgG class was further purified by adsorbing the fraction onto a Protein A Cellulofine (Seikagaku Kogyo) column equilibrated with 50 mM Tris-HCl buffer (pH 8.6) containing 0.15 M NaCl to remove the unadsorbed fraction and then eluted with 50 mM acetate buffer (pH 4.0) containing 0.15 M NaCl. The eluates were immediately neutralized with 1.5 M Tris-HCl buffer (pH 8.9).

Example 2

Quantitation of the major central triple-helical region of type IV collagen in human sera
(a) Preparation of enzyme-labeled monoclonal antibody (Fab'-POD conjugate) :
    (1) Preparation of Fab' fraction
        The IgG fraction obtained in Example 1 (j) was dissolved in 0.1 M acetate buffer (pH 4.2), containing 0.1 M NaCl and the solution was digested with pepsin as follows. Thus, pepsin was added to the fraction to 2 % (w/w) based on the IgG therein, and the mixture was subjected to digestion at 37°C for 24 hrs. The digested product was adjusted to a pH of 7.0 with 2 M Tris solution thereby to stop the digestion reaction. The product was gel-filtered on a Ultrogel AcA 44 column (LKB) equilibrated with 0.1 M phosphate buffer (pH 7.0) to obtain $F(ab')_2$ fraction.
        The $F(ab')_2$ fraction was dialyzed against 0.1 M phosphate buffer (pH 6.0) containing 5 mM ethylenediamine tetraacetic acid (EDTA), and aminoethanethiol (MEA) was added to a final concentration of 10 mM to effect reduction at 37°C for 1.5 hrs. The product was gel-filtered on a Ultrogel AcA 44 column equilibrated with 0.1 M phosphate buffer (pH 6.0) containing 5 mM EDTA to obtain Fab' fraction.
    (2) Preparation of maleimide-labeled POD fraction
        Separately from the procedure as mentioned in (1) above, POD was labeled with a maleimide compound as follows. Thus, the POD was dissolved in 0.1 M phosphate buffer (pH 7.0) to 10 mg/ml and to the solution was added 25 moles, per mole of the POD, of N-(ε-maleimidocaproyloxy)succinimide (EMCS) as a solution in dimethylformamide for reaction at 30°C for 30 min. The product was gel-filtered on a Sephadex G-50 column equilibrated with 0.1 M phosphate buffer solution (pH 6.0) to obtain maleimide-labeled POD fraction.
    (3) Preparation of Fab'-POD conjugate fraction
        The fractions as prepared in (1) and (2) above were admixed so that equimolar amounts of Fab' and maleimide-labeled POD in the respective fractions were mixed. The mixture was then diluted with 0.1 M phosphate buffer (pH 6.0) containing 5 mM EDTA to a final concentration, in respect of both Fab' and maleimide-labeled POD, of 100 μM. After reaction at 4°C for 20 hrs. was added to 10 moles, per mole of Fab', of N-ethylmaleimide to block unreacted thiol groups. The product was gel-filtered on a Ultrogel AcA 44 column equilibrated with 0.1 M phosphate buffer (pH 6.5) to obtain Fab'-POD conjugate fraction, to which 0.1 % BSA and 0.005 % thimerosal were added for storage at 4°C.
(b) One step sandwich technique using a polystyrene ball (6.5 mm in diameter, Precision Plastic Ball) as a solid phase carrier:
    The purified monoclonal antibody against pepsin-solubilized human type IV collagen obtained in Example 1 (j) (Clone No. 4H12) was dissolved in 0.1 M phosphate buffer (pH 7.5) containing 0.1 % sodium azide to a concentration of 0.1 mg/ml. Polystyrene balls as solid phase carrier were soaked in this antibody solution so that the polystyrene balls were coated with the antibody. The antibody solution used for soaking was then recovered and the polystyrene balls were washed with 10 mM phosphate buffer (pH 7.0) containing 0.1 % BSA, 0.1 M NaCl and 0.1 % sodium azide, and stored at 4°C. In its actual use, the antibody-polystyrene balls were washed with 10 mM phosphate buffer (pH 7.0) containing 0.1 M NaCl.
    As a standard sample was used the purified pepsin-solubilized human type IV collagen obtained in Example 1 (a). Thus a 40 ng/20 μl solution thereof in 10 mM phosphate buffer (pH 7.0) containing 1 % BSA, 0.1 M NaCl, 1/50 (v/v) horse serum (M.A. Bioproducts) and 0.05 % thimerosal was prepared and the solution was diluted stepwise. A 20 μl aliquot from each dilution was taken as a sample to be assayed.
    As serum samples were used 20 μl of serum from each normal subject (NOR) and 20 μl of serum from each patient with hepatocellular carcinoma (HCC), with liver cirrhosis (LC) or with chronic active hepatitis (CAH). These samples were each dissolved in 300 μl of 10 mM phosphate buffer (pH 7.0) containing 0.8 μg/ml Fab' (Clone No. 1D3)-POD conjugate, 1 % BSA, 0.1 M NaCl, 1/50 (v/v) horse serum and 0.05 % thimerosal. To each of these standard samples and serum samples was added the monoclonal antibody-coated polystyrene ball. The system was incubated at 37°C for 45 min. (immunoreaction) and the ball was washed with 10 mM phosphate buffer (pH 7.0) containing 0.1 M NaCl. To each ball was then added 300 μl of a solution of a substrate for POD in 0.1 M acetate buffer (pH 5.5), i.e. 0.0134 % tetramethylbenzidine (TMBZ). After further addition of 100 μl of 0.01 % aqueous $H_2O_2$, the system was incubated at 37°C for 30 min. (coloring reaction) and 600 μl of 1.33 N sulfuric acid was added to stop the reaction. After the stop of the reaction, the absorbance was measured at 450 nm by means of a Shimazu microflow ultraviolet-visible spectrophotometer (UV-730), using water as a reference, and a difference between the absorbance of the blank and that of the sample was calculated. From a standard curve prepared with the standard sample the amount of type IV collagen peptide which corresponds to the absorbance of 20 μl of the sample was read out and multiplied by 50 to give the amount of type IV collagen contained in 1 ml of the sample (Table 2).

Table 2

| | Sample | Standard solution for preparing a standard curve | Reagent blank |
|---|---|---|---|
| Sample | 20 μl | 20 μl | |
| Enzyme-labeled antibody solution | 300 μl | 300 μl | |
| Antibody-coated ball | 1 ball | 1 ball | |
| [Immunoreaction] | Mixing followed by standing and warming at 37˚C exactly for 45 min. | | |
| [Washing] | Washed three times with 3 ml washing solution under suction with an aspirator | | |
| Substrate solution | 300 μl | 300 μl | 300 μl |
| Coloring subsidiary | 100 μl | 100 μl | 100 μl |
| [Coloring reaction] | Standing and warming at 37˚C exactly for 30 min. | | |
| Reaction stopper | 600 μl | 600 μl | 600 μl |
| [Colorimetry] | Measurement of absorbance at 450 nm | | |

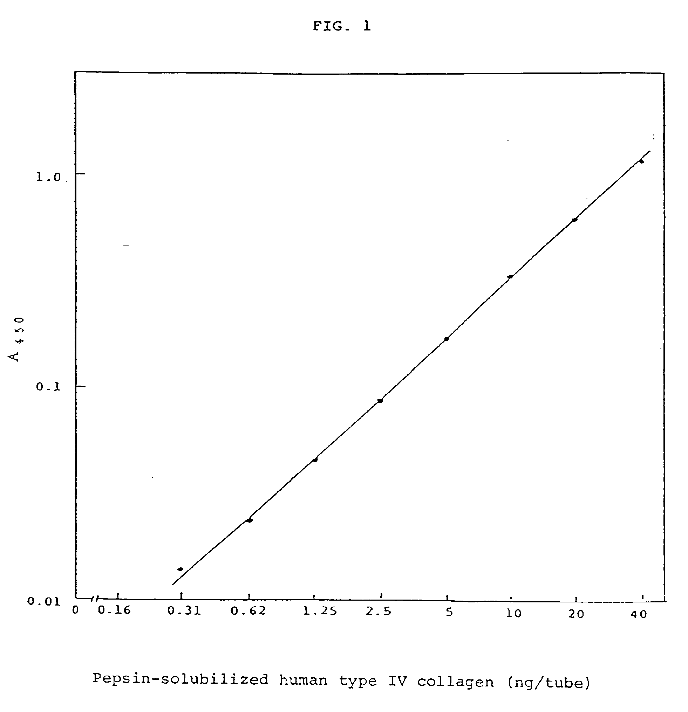

Fig. 1 shows the standard curve for pepsin-solubilized human type IV collagen. As is apparent from Fig. 1, the sensitivity of this sandwich technique was 0.31 ng/tube, and the linearity was obtained 0.31 - 40 ng/tube. The assay coefficient of variation (CV) was 2.6 - 9.5 %. The reaction times needed in this bead method were 45 min. for the immunoreaction and 30 min. for the coloring reaction, it thus being possible to make determinations within time periods shorter than half the time needed for conventional two step sandwich technique (for example Japanese Laid-Open Patent Appln. No. Sho-61-202162).

This sandwich technique was used to determine type IV collagen in sera from NOR and from HCC, LC and CAH patients, with the result that when M (mean) + 2SD (standard deviation) for NOR sera was taken as the upper reference value, the positivity for the liver disorders was 97 %, 80 % and 80 %, respectively (Fig. 2).

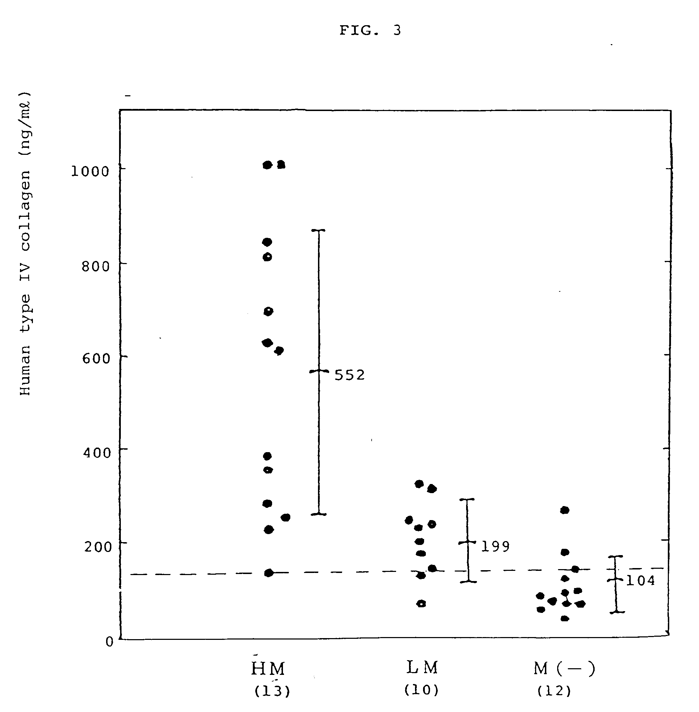

For a metastasis-negative group (M(-)) consisting of 12 patients with gastric cancer showing no metastasis, a liver metastasis group (HM) consisting of 13 such cases confirmed histologically or by diagnostic imaging and a limphogenous metastasis group (LM) of ten such cases, serum levels of type IV collagen were determined by this sandwich technique. As is apparent from Fig. 3, the type IV collagen levels for HM, LM and M(-) were $552 \pm 300$, $199 \pm 81$ and $104 \pm 62$ ng/ml ($M \pm SD$), respectively. When M + 2SD for NOR sera was taken as the upper reference value, the positivity for these disorders was 100 %, 80 % and 25 %, respectively.

(c) One step sandwich technique using a polystyrene microplate (Nunc) as a solid phase carrier:

The purified monoclonal antibody against pepsin-solubilized human type IV collagen obtained in Example 1 (j) (Clone No. 4H12) was dissolved in 0.1 M phosphate buffer (pH 7.5) containing 0.1 % sodium azide to a concentration of 0.1 mg/ml. To a polystyrene microplate as solid phase carrier was added 100 μl/well of this antibody solution to coat the plate therewith, and the thus coated plate was then stored at 4°C. In its actual use, the plate was blocked with 300 μl of 10 mM phosphate buffer (pH 7.0) containing 1 % BSA, 0.1 M NaCl and 0.1 % sodium azide, and then washed with 10 mM phosphate buffer (pH 7.0) containing 0.1 M NaCl and 0.1 % (v/v) Tween 20.

As a standard sample was used the purified pepsin-solubilized human type IV collagen obtained in Example 1 (a). Thus a 50 ng/50 μl solution thereof in 10 mM phosphate buffer (pH 7.0) containing 1 % BSA, 0.1 M NaCl, 1/50 (v/v) horse serum and 0.05 % thimerosal was prepared and the solution was diluted stepwise. A 20 μl aliquot from each dilution was applied to each well. As serum samples were used 20 μl/well of serum from each normal subject (NOR) and 20 μl/well of serum from each patient with HCC, LC, CAH or chronic inactive hepatitis (CIH). Thus, a 50 μl aliquot of each sample was dissolved in 200 μl of 10 mM phosphate buffer (pH 7.0) containing 0.8 μg/ml Fab' (Clone No. 1D3)-POD conjugate, 1 % BSA, 0.1 M NaCl, 1/50 (v/v) horse serum and 0.05 % thimerosal. A 100 μl/well aliquot of each such sample solution was added to the monoclonal antibody-coated microplate. The system was incubated at room temperature (10 - 30°C) for 1 hr. (immunoreaction). To each well was then added 100 μl of 10 mM phosphate buffer (pH 7.0) containing 0.1 M NaCl and 0.1 % (v/v) Tween 20 to stop the immunoreaction. Each well was then washed with 10 mM phosphate buffer (pH 7.0) containing 0.1 M NaCl and 0.1 % (v/v) Tween 20. OPD·2HCl was dissolved in citrate-phosphate buffer (pH 6.0) containing 0.02 % $H_2O_2$ to a concentration of 4 mg/ml, and 100 μl of the resultant solution (pH 5.0) was added thereto. The system was then incubated at room temperature for 15 min. (coloring reaction) and 100 μl of 1.33 N sulfuric acid was added to stop the reaction. After the stop of the reaction, the absorbance was measured at 492 nm by means of a microplate reader and a difference between the absorbance of the blank and that of the sample was calculated. From a standard curve prepared with the standard sample the amount of type IV collagen peptide which corresponds to the absorbance of 20 μl of the sample was read out and multiplied by 50 to give the amount of type IV collagen contained in 1 ml of the sample (Table 3).

Table 3

| | Sample | Standard solution for preparing a standard curve |
|---|---|---|
| Sample | 50 μl | 50 μl |
| Enzyme-labeled antibody solution | 200 μl | 200 μl |
| Antibody-coated microplate | 2 wells | 2 wells |
| [Immunoreaction] | Mixing and addition of 100 μl/well the mixed solution followed by standing at room temperature exactly for 60 min. | |
| Washing solution | 100 μl | 100 μl |
| [Washing] | Washed four times with 300 μl washing solution under suction with an aspirator | |
| Substrate solution | 100 μl | 100 μl |
| [Coloring reaction] | Standing at room temperature exactly for 15 min. | |
| Reaction stopper | 100 μl | 100 μl |
| [Colorimetry] | Measurement of absorbance at 492 nm | |

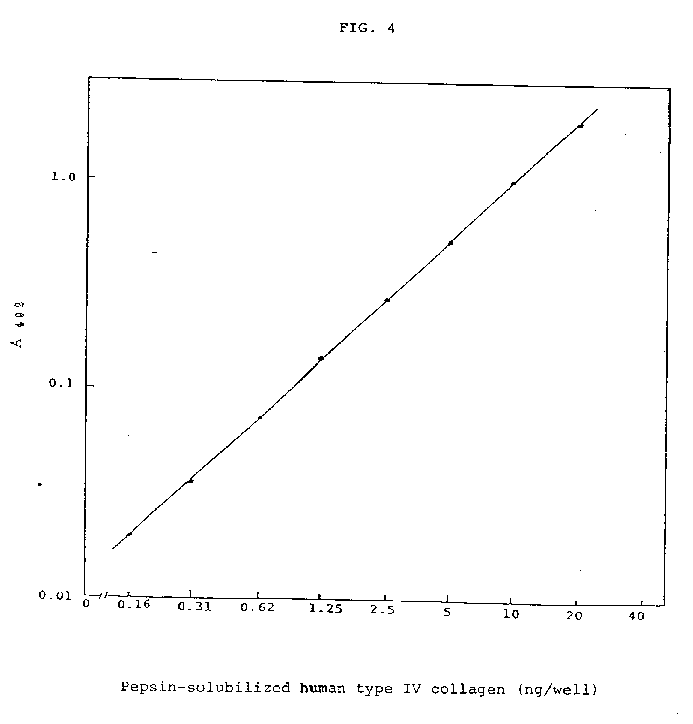

Fig. 4 shows the standard curve for pepsin-solubilized human type IV collagen. As is apparent from this figure, the sensitivity of this sandwich technique was 0.16 ng/well, and the linearity was 0.16 - 20 ng/well. The CV of this assay system was 0.5 - 6.0%. The reaction times needed in this plate method were 60 min. for the immunoreaction and 15 min. for the coloring reaction, it thus being possible to make determinations of a number of samples within time periods shorter than half the time needed for conventional two step sandwich technique. Very satisfactory results were obtained also in respect of precision when repeated determinations in intra-assay of the different samples and in inter-assay of the same sample were made (CV< 5 % for both cases), as shown in Table 4.

Table 4

(A) Intra-assay variations

| Sample No. | Human type IV collagen (ng/ml) | | |
|---|---|---|---|
| | A | B | C |
| 1 | 123 | 175 | 300 |
| 2 | 120 | 170 | 265 |
| 3 | 117 | 165 | 268 |
| 4 | 120 | 170 | 275 |
| 5 | 120 | 163 | 265 |
| 6 | 129 | 168 | 283 |
| 7 | 112 | 173 | 260 |
| 8 | 112 | 170 | 268 |
| M | 119 | 169 | 273 |
| SD | 5.6 | 3.9 | 13.0 |
| CV(%) | 4.7 | 2.3 | 4.8 |

EP 0 401 370 B1

(B) Inter-assay variations

| Day of determination / Sample | Human type IV collagen (ng/ml) | |
|---|---|---|
| | 1st day | 2nd day |
| 1 | 112 | 128 |
| 2 | 334 | 332 |
| 3 | 263 | 273 |
| 4 | 125 | 120 |
| 5 | 183 | 175 |
| 6 | 155 | 172 |
| 7 | 124 | 119 |
| 8 | 118 | 117 |
| 9 | 111 | 113 |
| 10 | 98 | 103 |
| CV (%) | 3.2 | |

When the method of this invention was used to quantitate collagen peptide, there were observed significant increases in collagen peptide-levels in sera from patients with HCC, LC and CAH. When M + 2SD for NOR sera was taken as the upper reference value, the positivity for each disorder was 92 % (HCC), 87 % (LC), 83 % (CAH) and 39 % (CIH), respectively (Fig. 5). Such increases in collagen peptide levels in sera from patients with liver disorders were in line with the tendency as observed when the one step sandwich technique was applied using a polystyrene ball as the solid phase carrier.

Example 3

(a) Identification of antigen:

The pepsin-solubilized human type IV collagen purified in Example 1 (a) was subjected to SDS-PAGE and then to Western blotting in accordance with the method of Tanabe as described in "Saibo Kogaku" (Cell Technology) 1 & 2, pp. 1061-1068 (1983), using the Fab′-POD conjugate obtained in Example 2 (a), whereby patterns of enzyme-antibody staining were obtained.

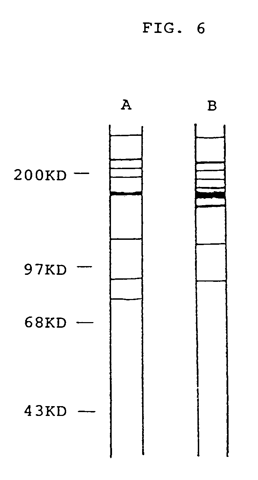

In patterns obtained by the SDS-PAGE followed by staining of proteins with Coomassie Brilliant Blue as well as in those obtained by subjecting each nitrocellulose membrane to the Western blotting followed by immunological staining with the Fab′ (clone No. 1D3)-POD conjugate as obtained in Example 2 (a) were observed five bands corresponding to 190 KD, 175 KD, 125 KD, 94 KD and 86 KD as well as two aggregates above 200 KD (205 KD and 220 KD) in the presence of mercaptoethanol. In similar patterns obtained by immunological staining with the Fab′ (Clone No. 4H12)-POD conjugate were shown five bands corresponding to 185 KD, 170 KD, 155 KD, 120 KD and 90 KD as well as two aggregates above 200 KD (200 KD and 220 KD) as shown in Fig. 6.

(b) Molecular weigbt of immunoreactive protein in human sera:

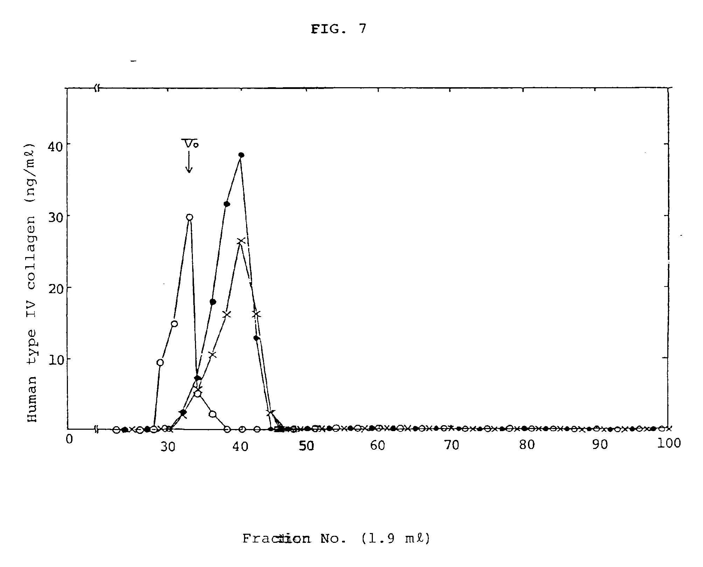

The molecular weight size was determined of the immunoreactive proteins captured by the enzyme immunoassays for human type IV collagen as described in Examples 2 (b) and (c). Thus, sera, each containing 300 ng of human type IV collagen, from NOR and patients with a HCC were gel-filtered using Sephacryl S-300 (1.6 x 90 cm, Pharmacia) equilibrated with 20 mM phosphate buffer (pH 7.0) containing 0.05 % Tween 20 and 0.15 M NaCl. Each fraction was subjected to the one step sandwich assay using polystyrene ball as described in Example 2 (b) to quantitate the immunoreactive proteins. As shown in Fig. 7, the immunoreactive proteins showed a single peak in the sera both from the NOR (-·-) and from patients with the liver disorder (-x-), and its molecular weight was somewhat smaller than that of pepsin-solubilized human type IV collagen peptide (300 ng, -o-), used as a reference. The average molecular weight of the immunoreactive proteins in the sera was estimated to be 620 KD from a standard curve prepared with fibrinogen, immunoglobulin, bovine serum albumin and peroxidase.

(c) Specificity:

The human type I, III, IV (acid extracted), V and VI collagens, 7-S domain, NC1 domain, laminin P1 fraction and pepsin-solubilized rat type IV collagen as prepared in Example 1 (b) were quantitated by the one step sandwich technique using polystyrene ball as described in Example 2 (b), for comparison with the value for the pepsin-solubilized human type IV collagen. As shown in Table 5, there was found no cross-reaction with any other collagen or laminin P1 fraction except for the type VI collagen, with which there was found slight cross-reaction (ca. 7%).

Table 5

Cross-reactivity with different types of collagen, type IV collagen 7-S domain, NC1 domain and laminin P1 fraction

| Concentration | Different types of collagen and basement membrane components | | | | | | | | |
|---|---|---|---|---|---|---|---|---|---|
| | I | III | IV* | IV** | V | VI | 7-S | NC1 | laminin P1 |
| 125 ng/ml (2.5 ng/tube) | 1.91 | 0.35 | 0.17 | 0 | 0 | 7.13 | 0 | 0.50 | 0 |

\* Acid-extracted type IV collagen,

\*\* Pepsin-solubilized rat type IV collagen

The figures in this table are expressed in terms of percentages relative to the value for the pepsin-solubilized human type IV collagen.

EP 0 401 370 B1

(d) Recovery (%):

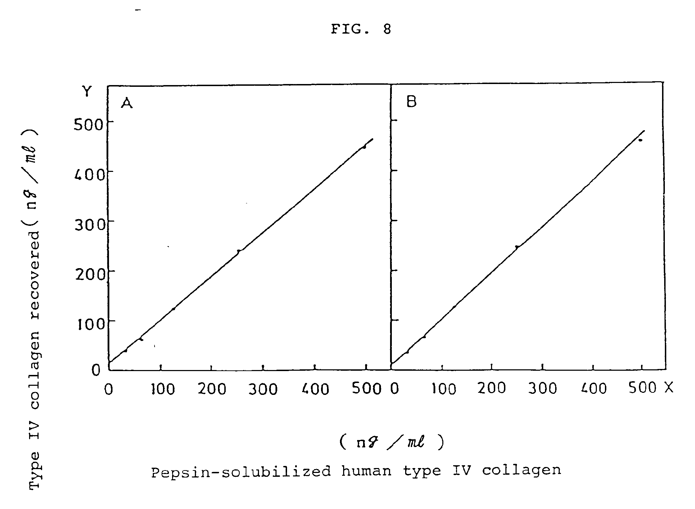

To two different human sera, i.e. one with a type IV collagen content of 93.0 ng/ml and the other with that of 234.0 ng/ml, was added pepsin-solubilized human type IV collagen at concentrations in the range of 31 - 500 ng/ml. Their recovery (%) was checked for by means of the one step sandwich technique using polystyrene ball or polystyrene plate as described in Example 2 (b) or 2 (c). As shown in Fig. 8, the coefficient of correlation and recovery (%) of the type IV collagen recovered relative to the pepsin-solubilized type IV collagen added were $\gamma = 0.9997$, $99.4 \pm 10.3$ % (M $\pm$ SD) and $\gamma = 0.9993$, $101.6 \pm 7.1$ % for the ball and plate methods, respectively.

(e) Antigenic determinants:

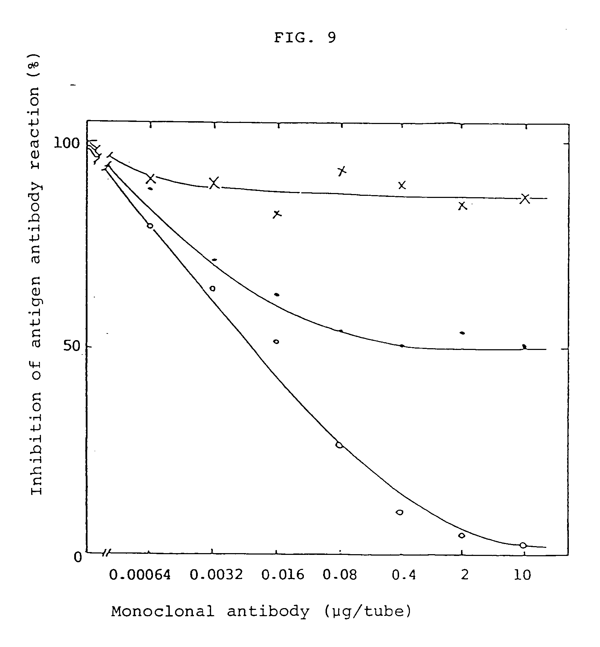

As indicated by the immunoblotting pattern shown in Fig. 6, the antigen recognizing sites of the solid phase antibody (Clone No. 4H12) and POD-labeled antibody (Clone No. 1D3) used in the one step sandwich technique using ball or plate as described in Examples 2 (b) and 2 (c) are slightly different. In order to elucidate the antigen recognizing sites of the solid phase antibody and POD-labeled antibody % inhibition of antigen antibody reaction was determined. Thus, 10 ng/tube of pepsin-solubilized human type IV collagen and 0 - 10 µg/tube of monoclonal antibody (Clone Nos. 1D3, 3A9 and 4H12) were preincubated at 37°C for 60 min. in 10 mM phosphate buffer (pH 7.0) containing 1 % BSA and 0.1 M NaCl. To each test tube were added a monoclonal antibody (Clone No. 4H12)-coated polystyrene ball prepared in accordance with the same procedure as in Example 2 (b) and 40 ng of Fab′ (Clone No. 3A9)-POD conjugate. After incubation at 37°C for 60 min. followed by addition of TMBZ and aqueous hydrogen peroxide, each test tube was allowed to stand at 30°C for 60 min., and then subjected to determination of $A_{450}$. As shown in Fig. 9, where the standard pepsin-solubilized human type IV collagen was treated with the monoclonal antibody from Clone No. 1D3 (-x-), no inhibition of antigen antibody reaction was observed. On the other hand where it was treated with the monoclonal antibodies from Clone No. 3A9 (---) and Clone No. 4H12 (-o-) used in the solid phase antibody and conjugate, there was observed an inhibition. These results clearly indicate that the solid phase antibody (Clone No. 4H12) used in the one step sandwich technique as disclosed herein reacts specifically with an antigenic determinant of pepsin-solubilized human type IV collagen which is different from that for the POD-labeled antibody (Clone No. 1D3).

Thus the results in (a) through (e) demonstrates that the one step sandwich enzyme immunoassay using ball or microplate as disclosed herein is an assay system capable of extremely specifically recognizing the major central triple-helical region of type IV collagen.

Example 4

(a) Preparation of anti human type IV collagen 7-S domain monoclonal antibodies:

Following the procedure as described in Example 1, 17 monoclonal antibodies against human type IV collagen 7-S domain were prepared. Four of these clones had immunoglobulin chains $\gamma 1/\kappa$ and the other 13 $\mu/\kappa$ (Table 6).

Table 6

| Clone No. | Isotype | Chain |
|---|---|---|
| 31-1G4 | IgG1 | $\gamma1/\kappa$ |
| 31-2H12 | IgM | $\mu/\kappa$ |
| 31-3D2 | IgG1 | $\gamma1/\kappa$ |
| 31-4H9 | IgM | $\mu/\kappa$ |
| 31-5H8 | IgM | $\mu/\kappa$ |
| 31-6H4 | IgM | $\mu/\kappa$ |
| 31-7E4 | IgM | $\mu/\kappa$ |
| 31-8G9 | IgG1 | $\gamma1/\kappa$ |
| 31-9H8 | IgM | $\mu/\kappa$ |
| 31-10F5 | IgM | $\mu/\kappa$ |
| 31-11D11 | IgM | $\mu/\kappa$ |
| 31-12G12 | IgM | $\mu/\kappa$ |
| 31-13H3 | IgG1 | $\gamma1/\kappa$ |
| 31-15B4 | IgM | $\mu/\kappa$ |
| 31-17H6 | IgM | $\mu/\kappa$ |
| 31-18H5 | IgM | $\mu/\kappa$ |
| 31-19F4 | IgM | $\mu/\kappa$ |

(b) One step sandwich technique using a polystyrene microplate (Nunc) as a solid phase carrier:

Purified monoclonal antibody against human type IV collagen 7-S domain as obtained in (a) above (Clone No. 31-8G9) was coated onto a microplate in the same manner as in Example 2 (c). POD-labeled antibody was prepared in the procedure as described in Example 2 using monoclonal antibody against pepsin-solubilized human type IV collagen peptide as obtained in Example 1 (Clone No. 4H12).

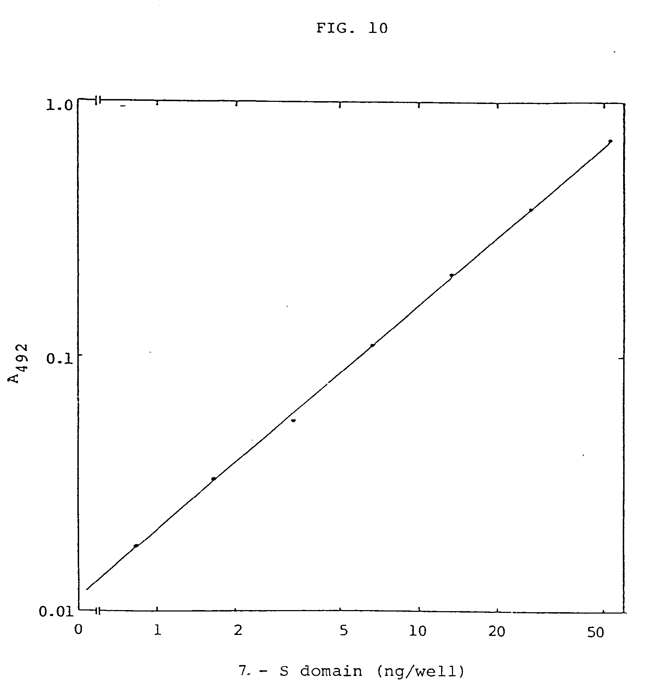

Using the human type IV collagen 7-S domain prepared in Example 1 (b) as a standard sample, a standard curve was prepared by the procedure shown in Table 7 (Fig. 10).

Table 7

| | Sample | Standard solution for preparing a standard curve |
|---|---|---|
| Sample | 100 μl | 100 μl |
| Enzyme-labeled antibody solution | 140 μl | 140 μl |
| Antibody-coated microplate | 2 wells | 2 wells |
| [Immunoreaction] | Mixing and addition of 100 μl/well the mixed solution followed by standing at room temperature exactly for 60 min. | |
| Washing solution | 100 μl | 100 μl |
| [Washing] | Washed five times with 300 μl washing solution under suction with an aspirator | |
| Substrate solution | 100 μl | 100 μl |
| [Coloring reaction] | Standing at room temperature exactly for 30 min. | |
| Reaction stopper | 100 μl | 100 μl |
| [Colorimetry] | Measurement of absorbance at 492 nm | |

As is apparent from Fig. 10, the sensitivity of this sandwich technique was 0.5 ng/well, and the linearity was obtained 0.5 - 54 ng/well. The CV in the assay was 0.3 - 6.8 %. The reaction times needed in this plate method were 60 min. for the immunoreaction and 30 min. for the coloring reaction, it thus being possible to make determinations of human type IV collagen 7-S domain within short assay time with high sensitivity as was the case with determinations of the major central triple-helical region of human type IV collagen as shown in Example 2 (c).

This sandwich technique was used to determine human type IV collagen 7-S domain in sera from NOR and from LC and HCC patients, with the result that when M + 2SD for NOR sera was taken as the upper reference value the positivity for LC and HCC was 71 % and 100 %, respectively (Table 8).

Table 8

| Sample No. | NOR sera (n=14) | LC sera (n=7) | HCC sera (n=7) |
|---|---|---|---|
| 1 | 6 ng/ml | 160 ng/ml | 166 ng/ml |
| 2 | 5 | 205 | 60 |
| 3 | 18 | 170 | 22 |
| 4 | 7 | 15 | 68 |
| 5 | 5 | 215 | 33 |
| 6 | 8 | 11 | 209 |
| 7 | 5 | 380 | 164 |
| 8 | 12 | | |
| 9 | 7 | | |
| 10 | 11 | | |
| 11 | 3 | | |
| 12 | 14 | | |
| 13 | 15 | | |
| 14 | 8 | | |
| M | 9 | 165 | 103 |
| SD | 4 | 127 | 75 |
| Positivity (%) | – | 71 | 100 |

(c) Recovery (%):

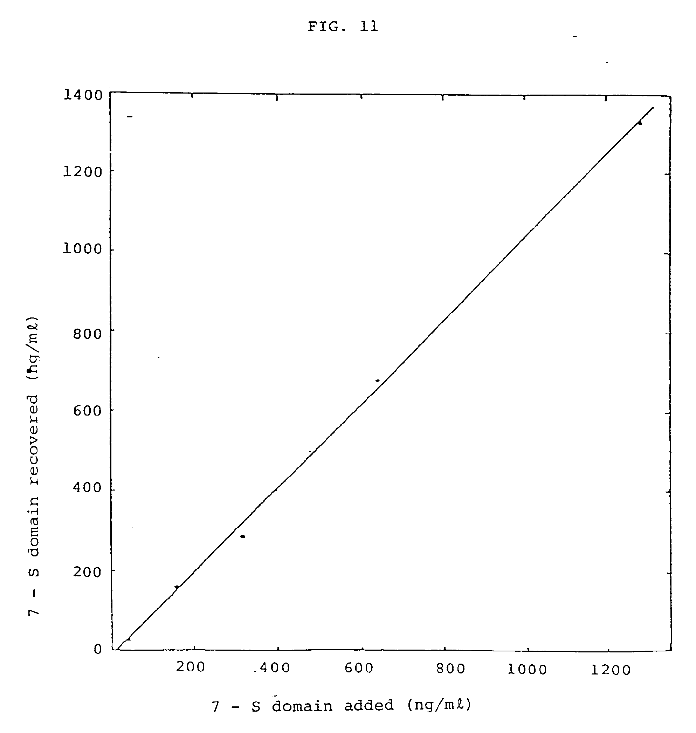

To a human serum with a type IV collagen 7-S domain content of 5 ng/ml was added human type IV collagen 7-S domain at concentrations in the range of 40 - 1280 ng/ml. Their recovery (%) was checked for by means of the one step sandwich technique using microplate as described in (b) above. As shown in Fig. 11, the coefficient of correlation and recovery (%) of the 7-S domain recovered relative to the 7-S domain added were $\gamma$ = 0.9991, and 94.2 ± 14.7 % (M ± SD). These results demonstrate that the one step sandwich technique using microplate as described in (b) above is a specific method of assaying for human type IV collagen 7-S domain.

BRIEF DESCRIPTION OF DRAWINGS

Fig. 1 shows a standard curve for pepsin-solubilized human type IV collagen by means of the one step sandwich technique using a polystyrene ball as a solid phase carrier (the bead method).

Fig. 2 shows serum levels determined by the ball method of immunoreactive type VI collagen peptide for NOR and for HCC, LC and CAH patients. In this figure, the horizontal bars indicate M, the broken line M + 2SD for NOR and each numeral in the parentheses the number of samples.

Fig. 3 shows serum levels determined by the bead method of immunoreactive type IV collagen for HM, LM and M(-) patients. In this figure, the vertical bars indicate M ± SD, the broken line M + 2SD and each numeral in the parentheses the number of samples.

Fig. 4 shows a standard curve for pepsin-solubilized human type IV collagen by means cf the one step sandwich technique using a polystyrene microplate as a solid phase carrier (the plate method).

Fig. 5 shows serum levels determined by the plate method of immunoreactive type IV collagen peptide for NOR and for HCC, LC, CAH and CIH patients. In this figure, the horizontal bars indicate M, the broken line M + 2SD and each numeral in the parentheses the number of samples.

Fig. 6 shows immunoblotting patterns of standard pepsin-solubilized human type IV collagen peptide. A : Stained with Fab′ (Clone No. 1D3)-POD conjugate; B: Stained with Fab′ (Clone No. 4H12)-POD conjugate.

Fig. 7 shows gel filtration patterns of immunoreactive proteins in human sera.

Fig. 8 shows correlations of the type IV collagen recovered to the pepsin-solubilized human type IV collagen peptide added to human sera for the ball method (A) and the plate method (B).

Fig. 9 shows % inhibition of antigen-antibody reaction for 3 kinds of monoclonal antibody.

Fig. 10 shows a standard curve for human type IV collagen 7-S domain.

Fig. 11 shows a correlation of the collagen 7-S domain recovered to the human type IV collagen 7-S domain added to human serum.

## Claims

1. A method for the quantitation of the major central triple-helical region of human type IV collagen by way of an enzyme immunoassay based on the sandwich technique using monoclonal antibodies against pepsin-solubilized human type IV collagen, in which

    (a) a sample solution prepared by diluting a sample to be assayed with a soluton in a buffer of an enzyme-labeled antibody obtained by labeling with an enzyme a monoclonal antibody which reacts with the major central triple-helical region of pepsin-solubilized human type IV collagen and

    (b) an antibody-coated solid phase comprising a monoclonal antibody, bound to a solid phase carrier, which reacts only with pepsin-solubilized human type IV collagen

    are used, and which comprises mixing the antibody-coated solid phase (b) with the solution (a) to cause an immunoreaction to occur among the human type IV collagen present in the said sample to be assayed, the said enzyme-labeled antibody and the antibody bound to the said solid phase carrier, isolating the solid phase carrier and measuring the enzyme activity bound to the solid phase thereby to quantitate the major central triple-helical region of human type IV collagen.

2. A method for the quantitation of human type IV collagen 7-S domain by an enzyme immunoassay based on the sandwich technique using monoclonal antibodies against human type IV collagen 7-S domain, in which

    (a) a sample solution prepared by diluting a sample to be assayed with a solution in a buffer of an enzyme-labeled antibody obtained by labeling with an enzyme a monoclonal antibody which reacts with pepsin-solubilized human type IV collagen 7-S domain and

    (b) an antibody-coated solid phase comprising a monoclonal antibody, bound to a solid phase carrier, which reacts only with human type IV collagen 7-S domain

    are used, and which comprises mixing the antibody-coated solid phase (b) with the solution (a) to cause an immunoreaction to occur among the human type IV collagen 7-S domain present in the said sample to be assayed, the said enzyme-labeled antibody and the antibody bound to the said solid phase carrier, isolating the solid phase carrier and measuring enzyme activity bound to the solid phase thereby to quantitate the human type IV collagen 7-S domain.

## Patentansprüche

1. Verfahren zur Quantifizierung des großen, zentralen, tripelhelicalen Bereichs des menschlichen Typ IV-Kollagens mittels eines auf dem Sandwich-Verfahren basierenden Enzymimmuntests unter Verwendung monoclonaler Antikörper gegen Pepsin-solubilisiertes menschliches Typ IV-Kollagen, wobei

    (a) eine Probenlösung, hergestellt durch Verdünnen einer zu untersuchenden Probe mit einer Lösung eines Enzym-markierten Antiköroers in einem Puffer, wobei der Antikörper erhalten wird, indem mit einem Enzym ein monoclonaler Antikörper markiert wird, der mit dem großen, zentralen, tripelhelicalen Bereich von Pepsin-solubilisiertem menschlichem Typ IV-Kollagen reagiert, und

    (b) eine Antikörper-überzogene feste Phase, die einen an einen Festphasen-Träger gebundenen monoclonalen Antikörper umfaßt, der nur mit Pepsin-solubilisiertem menschlichem Typ IV-Kollagen reagiert,

verwendet werden, und wobei die Antikörper-überzogene feste Phase (b) mit der Lösung (a) gemischt wird, wodurch eine Immunreaktion zwischen dem in der zu untersuchenden Probe vorhandenen menschlichen Typ IV-Kollagen, dem Enzym-markierten Antikörper und dem an den Festphasen-Träger gebundenen Antikörper auftritt, der Festphasen-Träger isoliert und die an die feste Phase gebundene Enzymaktivität gemessen wird, um so den großen, zentralen, tripelhelicalen Bereich des menschlichen Typ IV-Kollagens zu quantifizieren.

2. Verfahren zur Quantifizierung der 7-S-Domäne menschlichen Typ IV-Kollagens mittels eines auf dem Sandwich-Verfahren basierenden Enzymimmuntests unter Verwendung monoclonaler Antikörper gegen die 7-S-Domäne menschlichen Typ IV-Kollagens, wobei

(a) eine Probenlösung, hergestellt durch Verdünnen einer zu untersuchenden Probe mit einer Lösung eines Enzym-markierten Antikörpers in einem Puffer, wobei der Antikörper erhalten wird, indem mit einem Enzym ein monoclonaler Antikörper markiert wird, der mit der Pepsin-solubilisierten 7-S-Domäne menschlichen Typ IV-Kollagens reagiert, und

(b) eine Antikörper-überzogene feste Phase, die einen an einen Festphasen-Träger gebundenen monoclonalen Antikörper umfaßt, der nur mit der 7-S-Domäne menschlichen Typ IV-Kollagens reagiert,

verwendet werden, und wobei die Antikörper-überzogene feste Phase (b) mit der Lösung (a) gemischt wird, wodurch eine Immunreaktion zwischen der in der zu untersuchenden Probe vorhandenen 7-S-Domäne menschlichen Typ IV-Kollagens, dem Enzym-markierten Antikörper und dem an den Festphasen-Träger gebundenen Antikörper auftritt, der Festphasen-Träger isoliert und die an die feste Phase gebundene Enzymaktivität gemessen wird, um so die 7-S-Domäne menschlichen Typ IV-Kollagens zu quantifizieren.

## Revendications

1. Procédé pour la quantification de la grande région à triple hélice centrale du collagène humain de type IV, au moyen d'une analyse immunologique enzymatique reposant sur la technique en sandwich en utilisant des anticorps monoclonaux contre le collagène humain de type IV, solubilisé par la pepsine, dans lequel

(a) on prépare une solution d'échantillon en diluant un échantillon à analyser avec une solution dans un tampon d'un anticorps marqué à l'enzyme obtenu en marquant à l'aide d'une enzyme un anticorps monoclonal qui réagit avec la grande région à triple hélice centrale du collagène humain de type IV, solubilisé par pepsine, et

(b) une phase solide revêtue d'anticorps comprenant un anticorps monoclonal, lié à un support de phase solide qui réagit uniquement avec le collagène humain de type IV solubilisé par pepsine

sont utilisés, et qui comprend l'opération consistant à mélanger la phase solide revêtue d'anticorps (b) avec la solution (a) pour provoquer une immunoréaction parmi le collagène humain de type IV présent dans l'échantillon à analyser, l'anticorps marqué par enzyme et l'anticorps lié au support de phase solide, en isolant le support de phase solide et en mesurant l'activité enzymatique liée à la phase solide pour quantifier la grande région à triple hélice centrale du collagène humain de type IV.

2. Procédé pour la quantification de collagène humain de type IV domaine 7-S, par une analyse immunologique enzymatique reposant sur la technique en sandwich en utilisant des anticorps monoclonaux contre le collagène humain de type IV domaine 7-S, dans lequel on utilise,

(a) une solution d'échantillon préparée en diluant un échantillon à analyser avec une solution dans un tampon d'anticorps marqué par enzyme obtenu en marquant à l'aide d'une enzyme un anticorps monoclonal qui réagit avec le collagène humain de type IV, solubilisé par pepsine domaine 7-S, et

(b) une phase solide revêtue d'anticorps comprenant un anticorps monoclonal, lié à un support phase solide qui réagit uniquement avec le collagène humain de type IV, domaine 7-S, et qui comprend l'opération consistant à mélanger la phase solide revêtue d'anticorps (b) avec la solution (a) pour provoquer une immunoréaction parmi le collagène humain de type IV domaine 7-S, présent dans l'échantillon à analyser, l'anticorps marqué par enzyme et l'anticorps lié au support de phase solide, à isoler le support de phase solide et à mesurer l'activité enzymatique liée à la phase solide pour quantifier le collagène humain de type IV domaine 7-S.

FIG. 1

Pepsin-solubilized human type IV collagen (ng/tube)

FIG. 2

FIG. 3

FIG. 4

Pepsin-solubilized **human** type IV collagen (ng/well)

## FIG. 5

FIG. 6

FIG. 7

Fraction No. (1.9 mℓ)

27

FIG. 8

Type IV collagen recovered ( n𝓰 / m𝓵 )

( n𝓰 / m𝓵 )

Pepsin-solubilized human type IV collagen

FIG. 9

Monoclonal antibody (μg/tube)

FIG. 10

7. - S domain (ng/well)

FIG. 11